# EUROPEAN PATENT APPLICATION

(11) **EP 4 458 368 A1**
(43) Date of publication of application: **06.11.2024**
(21) Application number: 22914852.3
(22) Date of filing: 28.12.2022
(51) Int. Cl.: A61K 38/17, C07K 14/47, C12N 15/12, C12N 15/70, A61P 17/14, A61P 17/10

(54) **USE OF RECOMBINANT CALRETICULIN IN HAIR GROWTH, HAIR BREEDING, HAIR PROTECTION OR ANTI-HAIR LOSS AND RELATED PRODUCT**

(30) Priority: 30.12.2021 CN 202111668170
(71) Applicant: Beijing Yitubaihui Biotech Co., Ltd, Beijing 102308 (CN)
(72) Inventor: SUN, Chenglong, Beijing 102308 (CN); WEI, Dongdong, Beijing 102308 (CN); YU, Qian, Beijing 102308 (CN); WANG, Yi, Beijing 102308 (CN); HE, Gaiying, Beijing 102308 (CN); CUI, Junsheng, Beijing 102308 (CN); LI, Xiaoran, Beijing 102308 (CN)
(74) Representative: Puchberger & Partner Patentanwälte
(86) International application number: PCT/CN2022/142604
(87) International publication number: WO 2023/125600

(57) **Abstract**

Provided are a use of recombinant calreticulin in hair growth, hair breeding, hair protection or anti-hair loss and a related product. The recombinant protein is derived from the full length of calreticulin or a fragment thereof, and has the functions of treating hair loss and improving hair by means of injection or application. Therefore, calreticulin is expected to be developed into an effective method and means for treating hair loss and improving hair.

Sequence Listing
<110> BEIJING YITUBAIHUI BIOTECH CO., LTD
<120> USE OF RECOMBINANT CALRETICULIN IN HAIR GROWTH, HAIR BREEDING, HAIR PROTECTION OR
ANTI-HAIR LOSS AND RELATED PRODUCT
<160> 12
<170> SIPOSequenceListing 1.0
<210> 1
<211> 400
<212> PRT
<213> Artificial Sequence

<400> 1
Glu Pro Ala Val Tyr Phe Lys Glu Gln Phe Leu Asp Gly Asp Gly Trp
1               5                   10                  15
Thr Ser Arg Trp Ile Glu Ser Lys His Lys Ser Asp Phe Gly Lys Phe
            20                  25                  30
Val Leu Ser Ser Gly Lys Phe Tyr Gly Asp Glu Glu Lys Asp Lys Gly
        35                  40                  45
Leu Gln Thr Ser Gln Asp Ala Arg Phe Tyr Ala Leu Ser Ala Ser Phe
    50                  55                  60
Glu Pro Phe Ser Asn Lys Gly Gln Thr Leu Val Val Gln Phe Thr Val
65                  70                  75                  80
Lys His Glu Gln Asn Ile Asp Cys Gly Gly Gly Tyr Val Lys Leu Phe
                85                  90                  95
Pro Asn Ser Leu Asp Gln Thr Asp Met His Gly Asp Ser Glu Tyr Asn
            100                 105                 110
Ile Met Phe Gly Pro Asp Ile Cys Gly Pro Gly Thr Lys Lys Val His
        115                 120                 125
Val Ile Phe Asn Tyr Lys Gly Lys Asn Val Leu Ile Asn Lys Asp Ile
    130                 135                 140
Arg Cys Lys Asp Asp Glu Phe Thr His Leu Tyr Thr Leu Ile Val Arg
145                 150                 155                 160
Pro Asp Asn Thr Tyr Glu Val Lys Ile Asp Asn Ser Gln Val Glu Ser
                165                 170                 175
Gly Ser Leu Glu Asp Asp Trp Asp Phe Leu Pro Pro Lys Lys Ile Lys
            180                 185                 190
Asp Pro Asp Ala Ser Lys Pro Glu Asp Trp Asp Glu Arg Ala Lys Ile
        195                 200                 205
Asp Asp Pro Thr Asp Ser Lys Pro Glu Asp Trp Asp Lys Pro Glu His
    210                 215                 220
Ile Pro Asp Pro Asp Ala Lys Lys Pro Glu Asp Trp Asp Glu Glu Met
225                 230                 235                 240
Asp Gly Glu Trp Glu Pro Pro Val Ile Gln Asn Pro Glu Tyr Lys Gly
                245                 250                 255
Glu Trp Lys Pro Arg Gln Ile Asp Asn Pro Asp Tyr Lys Gly Thr Trp
            260                 265                 270

Ile His Pro Glu Ile Asp Asn Pro Glu Tyr Ser Pro Asp Pro Ser Ile
        275                 280                 285
Tyr Ala Tyr Asp Asn Phe Gly Val Leu Gly Leu Asp Leu Trp Gln Val
    290                 295                 300
Lys Ser Gly Thr Ile Phe Asp Asn Phe Leu Ile Thr Asn Asp Glu Ala
305                 310                 315                 320
Tyr Ala Glu Glu Phe Gly Asn Glu Thr Trp Gly Val Thr Lys Ala Ala
                325                 330                 335
Glu Lys Gln Met Lys Asp Lys Gln Asp Glu Glu Gln Arg Leu Lys Glu
            340                 345                 350
Glu Glu Glu Asp Lys Lys Arg Lys Glu Glu Glu Glu Ala Glu Asp Lys
        355                 360                 365
Glu Asp Asp Glu Asp Lys Asp Glu Asp Glu Glu Asp Glu Glu Asp Lys
    370                 375                 380
Glu Glu Asp Glu Glu Glu Asp Val Pro Gly Gln Ala Lys Asp Glu Leu
385                 390                 395                 400

<210>  2
<211>  1200
<212>  DNA
<213>  Artificial Sequence
<400>  2
gagcctgccg tctacttcaa ggagcagttt ctggacggag acgggtggac ttcccgctgg      60
atcgaatcca aacacaagtc agattttggc aaattcgttc tcagttccgg caagttctac     120
ggtgacgagg agaaagataa aggtttgcag acaagccagg atgcacgctt ttatgctctg     180
tcggccagtt tcgagccttt cagcaacaaa ggccagacgc tggtggtgca gttcacggtg     240
aaacatgagc agaacatcga ctgtgggggc ggctatgtga agctgtttcc taatagtttg     300
gaccagacag acatgcacgg agactcagaa tacaacatca tgtttggtcc cgacatctgt     360
ggccctggca ccaagaaggt tcatgtcatc ttcaactaca agggcaagaa cgtgctgatc     420
aacaaggaca tccgttgcaa ggatgatgag tttacacacc tgtacacact gattgtgcgg     480
ccagacaaca cctatgaggt gaagattgac aacagccagg tggagtccgg ctccttggaa     540
gacgattggg acttcctgcc acccaagaag ataaaggatc ctgatgcttc aaaaccggaa     600
gactgggatg agcgggccaa gatcgatgat cccacagact ccaagcctga ggactgggac     660
aagcccgagc atatccctga ccctgatgct aagaagcccg aggactggga tgaagagatg     720
gacggagagt gggaaccccc agtgattcag aaccctgagt acaagggtga gtggaagccc     780
cggcagatcg acaacccaga ttacaagggc acttggatcc acccagaaat tgacaacccc     840
gagtattctc ccgatcccag tatctatgcc tatgataact ttggcgtgct gggcctggac     900
ctctggcagg tcaagtctgg caccatcttt gacaacttcc tcatcaccaa cgatgaggca     960
tacgctgagg agtttggcaa cgagacgtgg ggcgtaacaa aggcagcaga gaaacaaatg    1020
aaggacaaac aggacgagga gcagaggctt aaggaggagg aagaagacaa gaaacgcaaa    1080
gaggaggagg aggcagagga caaggaggat gatgaggaca aagatgagga tgaggaggat    1140
gaggaggaca aggaggaaga tgaggaggaa gatgtccccg gccaggccaa ggacgagctg    1200

<210>  3
<211>  37
<212>  DNA

<213>  Artificial Sequence
<400>  3
ctctctcgag aaaagagagc ctgccgtcta cttcaag                                37

<210>  4
<211>  51
<212>  DNA
<213>  Artificial Sequence
<400>  4
gtaagcggcc gcctaatgat gatgatgatg atgcagctcg tccttggcct g                51

<210>  5
<211>  1200
<212>  DNA
<213>  Artificial Sequence
<400>  5
gaaccggcag tgtattttaa ggagcagttt ttagatggtg atggatggac cagtcggtgg       60
attgagagta agcataagag tgattttgga aaattcgttc tgagtagtgg gaaattttat      120
ggtgatgagg agaaggacaa gggactgcag acgagccagg atgcgcgctt ttatgcactg      180
agcgcatcat tcgaaccgtt tagcaataag ggtcagaccc tggtggtgca gtttacagtt      240
aaacatgagc agaatattga ctgtggtgga gggtatgtta agttatttcc gaatagctta      300
gaccagacgg atatgcatgg tgacagcgaa tacaacatca tgtttggacc cgatatttgt      360
ggtccgggta cgaagaaagt tcatgtgatt tttaattaca agggcaaaaa cgttctgatt      420
aacaaggata tacgttgtaa ggatgatgaa tttacccacc tgtataccct gattgtgcgt      480
cctgataata cctatgaggt taaaattgac aacagtcagg ttgagagcgg ttctctggaa      540
gacgattggg actttctgcc gccgaagaaa atcaaagatc cggacgcaag caaacctgaa      600
gactgggacg agagagcaaa aattgatgat ccgacagata gcaaaccgga agattgggat      660
aaaccggaac atatacctga cccggacgcc aaaaagccag aagattggga cgaagaaatg      720
gatggagaat gggaaccacc ggtgatacaa aatcctgaat acaaaggaga atggaaaccg      780
cgtcagattg acaatccgga ttataaaggc acatggatac atccggaaat agataatccg      840
gaatatagcc cggacccgtc tatttacgca tacgataatt ttggagtgct gggcttagat      900
ttatggcagg tgaaaagcgg cacaattttt gataattttc tgataaccaa cgacgaagca      960
tacgctgaag aatttggtaa cgaaacctgg ggtgtgacca aagcggctga aaaacagatg     1020
aaggataaac aggatgaaga acagcgcctt aaggaggagg aagaagacaa gaaacgcaaa     1080
gaggaggagg aggcagagga caaggaggat gatgaggaca aagatgagga tgaggaggat     1140
gaggaggaca aggaggaaga tgaggaggaa gatgtccccg gccaggccaa ggacgagctg     1200

<210>  6
<211>  30
<212>  DNA
<213>  Artificial Sequence
<400>  6
gttcatatgg aaccggcagt gtattttaag                                        30

<210>  7
<211>  33

<212>  DNA
<213>  Artificial Sequence
<400>  7
gtgctcgaga cgctgttctt catcctgttt atc                                    33

<210>  8
<211>  1047
<212>  DNA
<213>  Artificial Sequence
<400>  8
gaaccggcag tgtattttaa ggagcagttt ttagatggtg atggatggac cagtcggtgg      60
attgagagta agcataagag tgattttgga aaattcgttc tgagtagtgg gaaattttat     120
ggtgatgagg agaaggacaa gggactgcag acgagccagg atgcgcgctt ttatgcactg     180
agcgcatcat tcgaaccgtt tagcaataag ggtcagaccc tggtggtgca gtttacagtt     240
aaacatgagc agaatattga ctgtggtgga gggtatgtta agttatttcc gaatagctta     300
gaccagacgg atatgcatgg tgacagcgaa tacaacatca tgtttggacc cgatatttgt     360
ggtccgggta cgaagaaagt tcatgtgatt tttaattaca agggcaaaaa cgttctgatt     420
aacaaggata tacgttgtaa ggatgatgaa tttacccacc tgtataccct gattgtgcgt     480
cctgataata cctatgaggt taaaattgac aacagtcagg ttgagagcgg ttctctggaa     540
gacgattggg actttctgcc gccgaagaaa atcaaagatc cggacgcaag caaacctgaa     600
gactgggacg agagagcaaa aattgatgat ccgacagata gcaaaccgga agattgggat     660
aaaccggaac atatacctga cccggacgcc aaaaagccag aagattggga cgaagaaatg     720
gatggagaat gggaaccacc ggtgatacaa aatcctgaat acaaaggaga atggaaaccg     780
cgtcagattg acaatccgga ttataaaggc acatggatac atccggaaat agataatccg     840
gaatatagcc cggacccgtc tatttacgca tacgataatt ttggagtgct gggcttagat     900
ttatggcagg tgaaaagcgg cacaattttt gataattttc tgataaccaa cgacgaagca     960
tacgctgaag aatttggtaa cgaaacctgg ggtgtgacca aagcggctga aaaacagatg    1020
aaggataaac aggatgaaga acagcgc                                        1047

<210>  9
<211>  349
<212>  PRT
<213>  Artificial Sequence
<400>  9
Glu Pro Ala Val Tyr Phe Lys Glu Gln Phe Leu Asp Gly Asp Gly Trp
1               5                   10                  15
Thr Ser Arg Trp Ile Glu Ser Lys His Lys Ser Asp Phe Gly Lys Phe
            20                  25                  30
Val Leu Ser Ser Gly Lys Phe Tyr Gly Asp Glu Glu Lys Asp Lys Gly
        35                  40                  45
Leu Gln Thr Ser Gln Asp Ala Arg Phe Tyr Ala Leu Ser Ala Ser Phe
    50                  55                  60
Glu Pro Phe Ser Asn Lys Gly Gln Thr Leu Val Val Gln Phe Thr Val
65                  70                  75                  80
Lys His Glu Gln Asn Ile Asp Cys Gly Gly Gly Tyr Val Lys Leu Phe
                85                  90                  95

Pro Asn Ser Leu Asp Gln Thr Asp Met His Gly Asp Ser Glu Tyr Asn
            100                 105                 110
Ile Met Phe Gly Pro Asp Ile Cys Gly Pro Gly Thr Lys Lys Val His
        115                 120                 125
Val Ile Phe Asn Tyr Lys Gly Lys Asn Val Leu Ile Asn Lys Asp Ile
    130                 135                 140
Arg Cys Lys Asp Asp Glu Phe Thr His Leu Tyr Thr Leu Ile Val Arg
145                 150                 155                 160
Pro Asp Asn Thr Tyr Glu Val Lys Ile Asp Asn Ser Gln Val Glu Ser
                165                 170                 175
Gly Ser Leu Glu Asp Asp Trp Asp Phe Leu Pro Pro Lys Lys Ile Lys
            180                 185                 190
Asp Pro Asp Ala Ser Lys Pro Glu Asp Trp Asp Glu Arg Ala Lys Ile
        195                 200                 205
Asp Asp Pro Thr Asp Ser Lys Pro Glu Asp Trp Asp Lys Pro Glu His
    210                 215                 220
Ile Pro Asp Pro Asp Ala Lys Lys Pro Glu Asp Trp Asp Glu Glu Met
225                 230                 235                 240
Asp Gly Glu Trp Glu Pro Pro Val Ile Gln Asn Pro Glu Tyr Lys Gly
                245                 250                 255
Glu Trp Lys Pro Arg Gln Ile Asp Asn Pro Asp Tyr Lys Gly Thr Trp
            260                 265                 270
Ile His Pro Glu Ile Asp Asn Pro Glu Tyr Ser Pro Asp Pro Ser Ile
        275                 280                 285
Tyr Ala Tyr Asp Asn Phe Gly Val Leu Gly Leu Asp Leu Trp Gln Val
    290                 295                 300
Lys Ser Gly Thr Ile Phe Asp Asn Phe Leu Ile Thr Asn Asp Glu Ala
305                 310                 315                 320
Tyr Ala Glu Glu Phe Gly Asn Glu Thr Trp Gly Val Thr Lys Ala Ala
                325                 330                 335
Glu Lys Gln Met Lys Asp Lys Gln Asp Glu Glu Gln Arg
            340                 345

<210> 10
<211> 30
<212> DNA
<213> Artificial Sequence
<400> 10
gtgctcgagg tatgcgtaaa tagacgggtc 30

<210> 11
<211> 873
<212> DNA
<213> Artificial Sequence
<400> 11
gaaccggcag tgtattttaa ggagcagttt ttagatggtg atggatggac cagtcggtgg 60

attgagagta agcataagag tgattttgga aaattcgttc tgagtagtgg gaaattttat 120
ggtgatgagg agaaggacaa gggactgcag acgagccagg atgcgcgctt ttatgcactg 180
agcgcatcat tcgaaccgtt tagcaataag ggtcagaccc tggtggtgca gtttacagtt 240
aaacatgagc agaatattga ctgtggtgga gggtatgtta agttatttcc gaatagctta 300
gaccagacgg atatgcatgg tgacagcgaa tacaacatca tgtttggacc cgatatttgt 360
ggtccgggta cgaagaaagt tcatgtgatt tttaattaca agggcaaaaa cgttctgatt 420
aacaaggata tacgttgtaa ggatgatgaa tttacccacc tgtataccct gattgtgcgt 480
cctgataata cctatgaggt taaaattgac aacagtcagg ttgagagcgg ttctctggaa 540
gacgattggg actttctgcc gccgaagaaa atcaaagatc cggacgcaag caaacctgaa 600
gactgggacg agagagcaaa aattgatgat ccgacagata gcaaaccgga agattgggat 660
aaaccggaac atatacctga cccggacgcc aaaaagccag aagattggga cgaagaaatg 720
gatggagaat gggaaccacc ggtgatacaa aatcctgaat acaaaggaga atggaaaccg 780
cgtcagattg acaatccgga ttataaaggc acatggatac atccggaaat agataatccg 840
gaatatagcc cggacccgtc tatttacgca tac 873

<210> 12
<211> 291
<212> PRT
<213> Artificial Sequence
<400> 12
Glu Pro Ala Val Tyr Phe Lys Glu Gln Phe Leu Asp Gly Asp Gly Trp
1               5                   10                  15
Thr Ser Arg Trp Ile Glu Ser Lys His Lys Ser Asp Phe Gly Lys Phe
            20                  25                  30
Val Leu Ser Ser Gly Lys Phe Tyr Gly Asp Glu Glu Lys Asp Lys Gly
        35                  40                  45
Leu Gln Thr Ser Gln Asp Ala Arg Phe Tyr Ala Leu Ser Ala Ser Phe
    50                  55                  60
Glu Pro Phe Ser Asn Lys Gly Gln Thr Leu Val Val Gln Phe Thr Val
65                  70                  75                  80
Lys His Glu Gln Asn Ile Asp Cys Gly Gly Gly Tyr Val Lys Leu Phe
                85                  90                  95
Pro Asn Ser Leu Asp Gln Thr Asp Met His Gly Asp Ser Glu Tyr Asn
            100                 105                 110
Ile Met Phe Gly Pro Asp Ile Cys Gly Pro Gly Thr Lys Lys Val His
        115                 120                 125
Val Ile Phe Asn Tyr Lys Gly Lys Asn Val Leu Ile Asn Lys Asp Ile
    130                 135                 140
Arg Cys Lys Asp Asp Glu Phe Thr His Leu Tyr Thr Leu Ile Val Arg
145                 150                 155                 160
Pro Asp Asn Thr Tyr Glu Val Lys Ile Asp Asn Ser Gln Val Glu Ser
                165                 170                 175
Gly Ser Leu Glu Asp Asp Trp Asp Phe Leu Pro Pro Lys Lys Ile Lys
            180                 185                 190
Asp Pro Asp Ala Ser Lys Pro Glu Asp Trp Asp Glu Arg Ala Lys Ile
        195                 200                 205

Asp Asp Pro Thr Asp Ser Lys Pro Glu Asp Trp Asp Lys Pro Glu His
    210                 215                 220
Ile Pro Asp Pro Asp Ala Lys Lys Pro Glu Asp Trp Asp Glu Glu Met
225                 230                 235                 240
Asp Gly Glu Trp Glu Pro Pro Val Ile Gln Asn Pro Glu Tyr Lys Gly
                245                 250                 255
Glu Trp Lys Pro Arg Gln Ile Asp Asn Pro Asp Tyr Lys Gly Thr Trp
            260                 265                 270
Ile His Pro Glu Ile Asp Asn Pro Glu Tyr Ser Pro Asp Pro Ser Ile
        275                 280                 285
Tyr Ala Tyr
    290

## Description

### FIELD

The present disclosure relates to the field of pharmaceutical biotechnology, and in particular to use of recombinant calreticulin in hair growth, hair maintenance, hair protection or hair loss prevention and related products.

### BACKGROUND

Hair loss is a common clinical disease, and the cause of hair loss is not yet fully understood. Potential contributing factors include immune status, genetic causes, hormone levels, local inflammatory response, nerves and environmental factors. Common types of hair loss include androgenic alopecia (AGA), alopecia areata, telogen effluvium, senile alopecia, and others. Among these, AGA is the type of hair loss with the highest incidence. The literature indicates that the incidence of AGA in 30-year-old white men is 30%, the incidence of AGA in 50-year-old men is as high as 50%, and the incidence of AGA in men and women over 70 years old is 80% and 53%, respectively. The incidence of AGA in China is reported to be 21.3% for men and 6.0% for women. Finasteride and minoxidil are the only drugs approved by the US FDA for the treatment of hair loss. However, the clinical efficacy of these two drugs is not satisfactory, and they have certain side effects. Therefore, there is an urgent need for the development of safe and efficient new drugs and treatments.

Calreticulin (CRT) was initially identified within the endoplasmic reticulum (ER) of cells and exhibits high conservation during evolution. CRT, belonging to the heat shock protein family, is primarily located in the endoplasmic reticulum and performs a multitude of biological functions. CRT is a Ca²⁺ binding protein. The molecular weight of human CRT is about 46kDa, and it is divided into three domains: N, P, and C. The connection between the N and P regions is an important region for the protein to function as a molecular chaperone. The P region has a high affinity for calcium ions, yet the amount of binding is relatively small. The C region, in contrast, has a low affinity for calcium ions but a large capacity, and is considered to serve as a "calcium store" in the cell. Recent studies have demonstrated that CRT also exists in locations other than the endoplasmic reticulum and plays an important biological function, such as participating in antigen processing and presentation of adaptive immunity, anti-tumor immune response, mediating phagocytosis of apoptotic cells, cell adhesion and migration, and proliferation and activation of immune cells. Subsequent studies found that CRT is also present in the cell matrix and on the surfaces of almost all immune cells, including monocytes/macrophages, neutrophils, T cells, and dendritic cells, playing an important role in cell activation and the clearance of apoptotic cells and tumor cells. Calcium ions are important second messengers in cells. Changes in intracellular calcium ion concentrations have significant effects on numerous cellular functions, including the secretion of various cell active substances, muscle contraction and relaxation, protein modification, and gene expression. The endoplasmic reticulum is an important place for most proteins in their folding, modification, and assembly. Protein synthesis inevitably results in the formation of some misfolded proteins. In such circumstances, calreticulin acts as a molecular chaperone, facilitating the completion of protein repair in the presence of various enzymes. CRT can also bind to calnexin to jointly repair glycoproteins that are not synthesized normally.

To date, there is no published literature or related reports revealing the application of CRT in hair growth and improvement.

### SUMMARY

In view of the above-mentioned technical limitations, the present disclosure provides calreticulin, a DNA sequence encoding the protein, a vector or plasmid comprising the DNA sequence, a host cell, a method for preparing the protein by genetic engineering, and use of the protein in hair growth, hair maintenance, hair loss prevention, promotion or protection of hair growth, etc., overcoming the shortcomings and defects mentioned in the background.

To achieve the above objective, the present disclosure provides the following technical solutions:
The purpose of the present disclosure is to provide the calreticulin protein involved, a DNA sequence encoding the protein, a vector or plasmid comprising the DNA sequence, a host cell, a method for preparing the protein by genetic engineering, and application of the protein in hair growth, hair maintenance, hair loss prevention, promotion or protection of hair growth, etc.

In order to achieve the above object, the present disclosure adopts the following technical solutions:
The present disclosure discloses application of calreticulin (CRT) in preparing a product for hair growth, hair maintenance, hair protection or hair loss prevention.

Preferably, the calreticulin CRT is a protein of recombinantly expressed full-length CRT (1-400 aa), rh-CRT-1, and having an amino acid sequence set forth in SEQ ID NO: 1, or a protein having a similarity of at least 85% to the amino acid sequence set forth in SEQ ID NO: 1 and having a similar function.

The above-mentioned protein includes proteins with CRT-1 homologous sequences with similarities of 85%, 90%, 95%, and 99% (i.e., 340 aa, 360 aa, 380 aa, and 396 aa identical to the full-length CRT protein, respectively).

Preferably, the calreticulin CRT is a protein comprising a fragment (1-349 aa) of recombinantly expressed CRT, rh-CRT-2, and having an amino acid sequence set forth in SEQ ID NO: 9, or a protein having a similarity of at least 85% to the amino acid sequence set forth in SEQ ID NO: 9 and having a similar function

The above-mentioned protein includes proteins with CRT-2 homologous sequences with similarities of 86%, 89%, 95%, and 99% (i.e., 300 aa, 310 aa, 330 aa, and 345 aa identical to the 349 aa fragment of CRT protein, respectively).

Preferably, the calreticulin CRT is a protein comprising a fragment (1-291 aa) of recombinantly expressed CRT, rh-CRT-3, and having an amino acid sequence set forth in SEQ ID NO: 12, or a protein having a similarity of at least 85% to the amino acid sequence set forth in SEQ ID NO: 12 and having a similar function

The above-mentioned protein includes proteins with CRT-3 homologous sequences with similarities of 86%, 91%, 96%, and 99% (i.e., 250 aa, 265 aa, 280 aa, and 288 aa identical to the 291 aa fragment of CRT protein, respectively).

The present disclosure discloses a recombinant protein related to hair growth, hair maintenance, hair protection or hair loss prevention, wherein the recombinant protein is rh-CRT-1, rh-CRT-2 or rh-CRT-3; wherein rh-CRT-1 has an amino acid sequence set forth in SEQ ID NO: 1, rh-CRT-2 has an amino acid sequence set forth in SEQ ID NO: 9, and rh-CRT-3 has an amino acid sequence set forth in SEQ ID NO: 12.

Furthermore, the present disclosure discloses a gene encoding the recombinant protein related to hair growth, hair maintenance, hair protection and hair loss prevention, wherein the gene has a nucleotide sequence encoding rh-CRT-1 set forth in SEQ ID NO: 2, a nucleotide sequence encoding rh-CRT-2 set forth in SEQ ID NO: 8, or a nucleotide sequence encoding rh-CRT-3 set forth in SEQ ID NO: 11.

The present disclosure also discloses a plasmid comprising the gene encoding the recombinant protein related to hair growth, hair maintenance, hair protection and hair loss prevention.

The present disclosure also discloses a vector comprising the gene encoding the recombinant protein related to hair growth, hair maintenance, hair protection and hair loss prevention as an active ingredient.

The present disclosure discloses application of the above-mentioned recombinant protein, coding gene, plasmid or vector in the manufacture of a medicament or medical device product for hair growth, hair maintenance, hair protection or hair loss prevention.

Preferably, the medicament is utilized to improve the state of hair shaft and hair cuticle.

Preferably, the medicament is utilized to increase the number of skin hair follicles.

Preferably, the medicament promotes hair growth by reducing the content of androgen.

More preferably, the androgen is dihydrotestosterone.

The present disclosure also discloses a product with the functions of hair growth, hair maintenance, hair protection or hair loss prevention, comprising the above-mentioned recombinant protein, gene, plasmid or vector as an active ingredient;
wherein the product is utilized for at least one of the following:
   a) improving hair smoothness;
   b) increasing hair volume;
   c) promoting hair growth;
wherein the product is a medicament, an additive or an active ingredient.

The present disclosure also discloses a medicament with the functions of hair growth, hair maintenance, hair protection or hair loss prevention, comprising the above-mentioned recombinant protein, gene, plasmid or vector and a pharmaceutically acceptable adjuvant.

The medicament further comprises a pharmaceutical preparation that can be used in combination, preferably minoxidil.

Preferably, the adjuvant is selected from the group consisting of a diluent, excipient, filler, binder, wetting agent, disintegrant, absorption promoter, surfactant, adsorption carrier, lubricant, and a mixture thereof.

Preferably, the medicament is introduced into a body tissue by injection, spraying, penetration, absorption, or a physical or chemical method; or the medicament is introduced into a body after being mixed or encapsulated by other substances.

Preferably, the medicament is an external preparation.

The present disclosure also discloses application of calreticulin CRT in the manufacture of a medicament or medical device product for treating acne, pimples or alopecia areata.

Preferably, the calreticulin CRT is a protein of recombinantly expressed full-length CRT and having an amino acid sequence set forth in SEQ ID NO: 1, or a protein having a similarity of at least 85% to the amino acid sequence set forth in SEQ ID NO: 1 and having a similar function.

Preferably, the calreticulin CRT is a protein comprising a fragment of recombinantly expressed CRT and having an amino acid sequence set forth in SEQ ID NO: 9, or a protein having a similarity of at least 85% to the amino acid sequence set forth in SEQ ID NO: 9 and having a similar function.

Preferably, the calreticulin CRT is a protein comprising a fragment of recombinantly expressed CRT and having an amino acid sequence set forth in SEQ ID NO: 12, or a protein having a similarity of at least 85% to the amino acid sequence set forth in SEQ ID NO: 12 and having a similar function.

Compared with the prior art, the present disclosure has the following beneficial effects:

The present disclosure discloses for the first time the application of calreticulin (CRT) in hair growth, hair maintenance, hair protection or hair loss prevention. Further, the present disclosure discloses for the first time a recombinant protein with the above-mentioned functions of hair growth, hair maintenance, hair protection or hair loss prevention, and clarifies the preparation method and application of the recombinant protein. The protein is derived from calreticulin or its fragments, and the proteins having a sequence similarity of 95%, 90% or 85% with the above-mentioned recombinant protein and having the functions of hair growth, hair maintenance, hair protection or hair loss prevention. The present disclosure has been verified by sufficient experiments that the recombinant protein disclosed in the present disclosure has the function of treating hair loss and improving hair by injection or external application. Therefore, it is anticipated that the CRT protein will be developed as an efficacious method and means for the treatment of hair loss and improvement of hair.

### BRIEF DESCRIPTION OF DRAWINGS

FIG 1 is a plasmid map of pPICZaA-rh-CRT-1.
FIG 2 shows the electrophoresis results after digestion (Xho I/Not I) of plasmid pPICZαA-rh-CRT-1; molecular weight standard unit: bp.
FIG 3 shows the SDS-PAGE electrophoresis results of protein rh-CRT-1.
FIG 4 is a plasmid map of pET42a-rhCRT-2.
FIG 5 shows the electrophoresis results after digestion (Nde I/Xho I) of plasmid pET42a-rhCRT-2; molecular weight standard unit: bp.
FIG 6 shows the SDS-PAGE electrophoresis results of protein rh-CRT-2.
FIG 7 is a plasmid map of pET42a-rhCRT-3.
FIG 8 shows the electrophoresis results after digestion (Nde I/Xho I) of plasmid pET42a-rhCRT-3; molecular weight standard unit: bp.
FIG 9 shows the SDS-PAGE electrophoresis results of protein rh-CRT-3.
FIG 10 shows the normal control group (dermoscope).
FIG 11 shows the model group (dermoscope).
FIG 12 shows experimental group A (dermoscope).
FIG 13 shows experimental group B (dermoscope).
FIG 14 shows the positive control group - minoxidil (dermoscope).
FIG 15 shows the positive control group - finasteride (dermoscope).
FIG 16 shows the normal control group (scanning microscope).
FIG 17 shows the model group (scanning microscope).
FIG 18 shows experimental group A (scanning microscope).
FIG 19 shows experimental group B (scanning microscope).
FIG 20 shows the positive control group - minoxidil (scanning microscope).
FIG 21 shows the positive control group - finasteride (scanning microscope).
FIG 22 shows the hair growth of mice in each group.
FIG 23 shows mice in each group under dermoscope.
FIG 24 shows the color tester results of mice in each group.
FIG 25 shows the scanning electron microscope images of each experimental group.
FIG 26 shows the effects of the experimental groups on the number of hair follicles in mice with androgenic alopecia; (a) Pathological sections of hair follicles in mice with androgenic alopecia by experimental groups C and D; (b) Effects of experimental groups C and D on the number of hair follicles in mice with androgenic alopecia.
FIG 27 shows the effects of experimental groups C and D on serum and skin tissue hormones in mice with androgenic alopecia.
FIG 28 shows a comparison of the statistical results of the time and hair growth scores of mice in different drug administration experimental groups during the third experiment recorded in an embodiment of the present disclosure.
FIG 29 shows a comparison of the statistical results of the time and hair growth scores of mice in different drug administration experimental groups during the fourth experiment recorded in an embodiment of the present disclosure.

### DETAILED DESCRIPTION

In order to make the purpose, technical solution and advantages of the present disclosure more clear, the present disclosure is further described in detail below. However, it should be understood that the description herein is only utilized to illustrate the present disclosure and is not intended to limit the scope of the present disclosure.

Unless otherwise defined, all technical terms and scientific terms used herein have the same meaning as those commonly understood by those skilled in the art of the present disclosure, and the terms used in the specification of the present disclosure are only for the purpose of describing specific embodiments and are not intended to limit the present disclosure. The reagents and instruments used herein are all commercially available, and the characterization means involved can refer to the relevant descriptions in the prior art, which will not be repeated herein.

In order to further understand the present disclosure, the present disclosure is further described in detail below in conjunction with preferable embodiments.

### Example 1

### 1. Expression and purification of recombinant human calreticulin (rh-CRT)

The human calreticulin gene (CRT, GenBank: M84739.1) is 1958 bp and encodes 417 amino acids, of which amino acids 1-17 are signal peptides. The sequence of the mature polypeptide (amino acids 18-417) is set forth in SEQ ID NO: 1. The gene sequence of the mature polypeptide is set forth in SEQ ID NO: 2.

### 1.1 Cloning and expression of rh-CRT-1

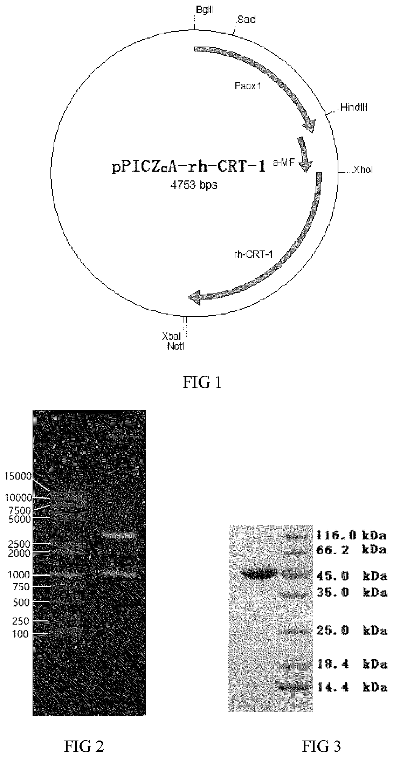

The amplification was carried out by PCR using the fully synthesized gene SEQ ID NO: 2 as template and pFcrt-1/pRcrt-I as upstream and downstream primers, wherein an XhoI restriction site was introduced upstream and a his-tag (his-his-his-his-his-his) sequence and a NotI restriction site were introduced downstream.
pFcrt-1 primer:
   5'-CTCTCTCGAGAAAAGAGAGCCTGCCGTCTACTTCAAG-3' (SEQ ID NO: 3);
pRcrt-1 primer:
   5' -GTAAGCGGCCGCCTAATGATGATGATGATGATGCAGCTCGTCCTTGGCCTG-3' (SEQ ID NO: 4);
The high-fidelity DNA polymerase Phusion from Thermo Scientific was used for PCR with 30 cycles including 98°C for 10 seconds, 55°C for 30 seconds, and 72°C for 60 seconds, followed by incubation at 72°C for 10 minutes. The resulting PCR product (about 1250 bp) was purified, digested with Xho I/Not I, and inserted into the plasmid pPICZαA (Invitrogen). The plasmid was then transformed into competent Escherichia coli TOP10, and positive clones were selected on LB medium containing the antibiotic Zeocin (Invitrogen). The plasmid was extracted and sequenced for verification. The plasmid was named pPICZαA-rh-CRT-1. The plasmid map is shown in FIG 1 and the enzyme digestion results are shown in FIG 2. After linearization by digestion with SacI, pPICZαA-rh-CRT-1 was transformed into Pichia pastoris GS115 by electroporation, and positive clones were selected on YPD plates containing the antibiotic Zeocin (100 µg/ml). 20 positive clones were selected, inoculated into small Erlenmeyer flasks containing 50 ml BMGY medium, and cultured at 30°C, 200 RPM until OD600 = 1.5. The yeast cells were collected by centrifugation, resuspended in 100 ml BMMY medium, transferred to a large Erlenmeyer flask of 500 ml, cultured at 30°C, 200 RPM, and then induced for expression using methanol with a final concentration of 0.5%. 1.0 ml of culture medium at 0 h, 24 h, 48 h, 72 h, and 96 h was subjected to centrifugation, and the expression level of the target protein in the supernatant was determined by SDS-PAGE. The clones with high expression were fermented in a 5L fermenter for 72h, and the supernatant was collected by centrifugation.

The culture media were prepared as follows:
LB liquid culture medium: 10.0 g peptone, 5.0 g yeast powder and 5.0 g sodium chloride were mixed, and purified water was added to 1.0 L. The pH was adjusted to 7.2. The medium was aliquoted in Erlenmeyer flasks, and sterilized at 121°C for 20 minutes.

LB solid culture medium: 10.0 g peptone, 5.0 g yeast powder, 5.0 g sodium chloride and 15.0 g agar powder were mixed, and purified water was added to 1.0 L. The pH was adjusted to 7.2. The medium was aliquoted in Erlenmeyer flasks, and sterilized at 121°C for 20 minutes.

YPD medium formula: 20.0 g peptone, 10.0 g yeast powder, 20.0 g glucose and 15.0 g agar powder were mixed, and purified water was added to 1.0 L. The medium was aliquoted in Erlenmeyer flasks, and sterilized at 115°C for 20 minutes.

BMGY medium: 20.0 g peptone, 10.0 g yeast powder, 11.8 g potassium dihydrogen phosphate, 3.0 g potassium dihydrogen phosphate trihydrate and 10.0 mL glycerol were mixed, and 900 mL purified water was added. The medium was sterilized at 115°C for 20 minutes. After cooling, 100 mL of 13.4% YNB solution after filtration and 2.0 mL of 0.02% biotin solution after filtration were added.

BMMY medium: 20.0 g peptone, 10.0 g yeast powder, 11.8 g potassium dihydrogen phosphate and 3.0 g potassium dihydrogen phosphate trihydrate were mixed, and 800 mL purified water was added. The medium was sterilized at 115°C for 20 minutes. After cooling, 100 mL of 13.4% YNB solution after filtration, 2.0 mL of 0.02% biotin solution after filtration, and 5.0 ml of 5.0% methanol solution after filtration were added.

### 1.2 Purification of rh-CRT-1 protein

1.0 L of the expression supernatant in the previous step was diluted with 1.5 L of 20 mM pH7.5 Tris-HCl buffer, and loaded with the AKTA purifier 100 system. The XK16/20 chromatography column and 20 ml of Ni sepharose 6FF from GE Company were used. After washing with 20 mM pH7.5 Tris-HCl buffer containing 100 mM imidazole, eluting with 25 mM EDTA solution, the protein peaks were collected. The protein peaks were concentrated with an ultrafiltration centrifuge tube with a molecular weight cutoff of 5 kDa, and loaded with the AKTA purifier 100 system. The XK16/100 chromatography column and Sephactyl S-200 from GE Company were used. The chromatography column was filled with a height of 95 cm. After eluting with 10 mM pH7.2 PB buffer containing 0.5% NaCl, the protein peaks were collected. The protein solution was rh-CRT-1, and the SDS-PAGE electrophoresis is shown in FIG 3.

### 1.3 Cloning and expression of rh-CRT-2

In order to express the protein in the prokaryotic system, the gene sequence of SEQ ID NO: 2 was codon optimized to a gene sequence set forth in SEQ ID NO: 5, and was subcloned (between BamH I/Xho I) into the pET28a plasmid (Novagen) by gene synthesis. The plasmid was named pET28a-MCRT.

The amplification was carried out by PCR using pET28a-MCRT as template and pFcrt-2/pRcrt-2 as upstream and downstream primers, wherein an Nde I restriction site was introduced upstream and an Xho I restriction site was introduced downstream.
pFcrt-2 primer 5'-GTTCATATGGAACCGGCAGTGTATTTTAAG-3' (SEQ ID NO: 6)
pRcrt-2p primer 5'-GTGCTCGAGACGCTGTTCTTCATCCTGTTTATC-3' (SEQ ID NO: 7)

The CRT gene sequence corresponding to rh-CRT-2 is set forth in SEQ ID NO: 8, and the protein sequence is set forth in SEQ ID NO: 9.

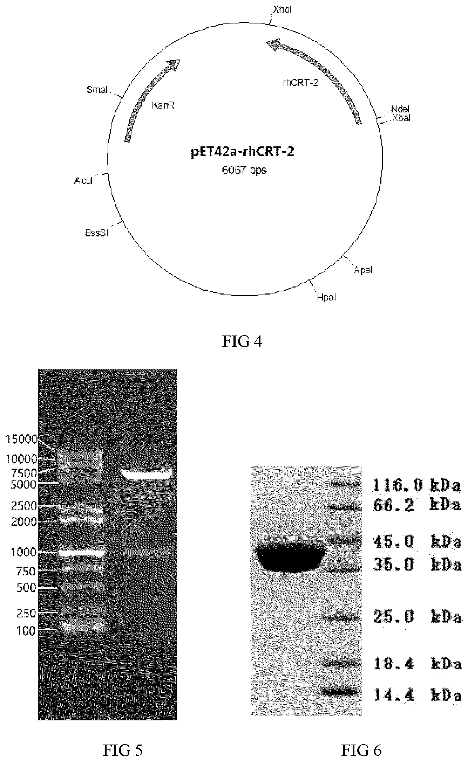

The Phusion high-fidelity DNA polymerase from Thermo Scientific was used for PCR with 30 cycles including 98°C for 10 seconds, 55°C for 30 seconds, and 72°C for 60 seconds, followed by incubation at 72°C for 10 minutes. The resulting PCR product (about 1060 bp) was purified, digested with Nde I/Xho I, and inserted into the plasmid pET42a (Novagen). The plasmid was then transformed into competent Escherichia coli TOP10, and positive clones were selected on LB medium containing kanamycin. The plasmid was extracted and sequenced for verification. The plasmid was named pET42a-rh-CRT-2. The plasmid map is shown in FIG 4 and the enzyme digestion results are shown in FIG 5. pET42a-rh-CRT-2 was transformed into competent BL21 (DE3) (Thermo Scientific). Positive clones were selected on LB plates containing kanamycin sulfate (50 µg/ml). The BL21 (DE3) positive clones obtained by transformation of pET42a-rh-CRT-2 were inoculated into 6.0ml LB liquid medium containing 50 µg/ml kanamycin sulfate, and cultured with shaking at 37°C, 220 RPM for about 3 hours. 2.0 ml bacterial liquid was inoculated in a 1.0 L Erlenmeyer flask containing 200 ml LB sterile medium with a final concentration of 50 µg/ml kanamycin sulfate, and cultured with shaking at 37°C, 220 RPM for 3 h until OD600 was about 1.0. Then, bacterial liquid was inoculated into six 5 L sterile Erlenmeyer flasks at a 1.0% inoculum, each of which contained 1.0 L LB medium, and cultured with shaking at 30°C, 220 RPM. When OD600 = about 0.8, 1.0 M IPTG (AMRESCO) was added to a final concentration of 0.5 mM, and cultured at a temperature adjusted to 28°C for 6 hours. Then the bacterial cells were collected by centrifugation.

### 1.4 Purification of rh-CRT-2 protein

10 g of the bacterial cells collected in 1.3 was added to 20 mM pH7.5 Tris-HCl buffer at a ratio of 1:20 (g/g) and subjected to ultrasonic lysis in an ice-water bath. The supernatant was collected by centrifugation, and loaded with an AKTA purifier 100 system. The XK16/20 chromatography column and 10 ml of Ni sepharose 6FF from GE Company were used. After washing with 20 mM pH7.5 Tris-HCl buffer containing 100 mM imidazole, eluting with 25 mM EDTA solution, the protein peaks were collected. The protein peaks were concentrated with an ultrafiltration centrifuge tube with a molecular weight cutoff of 5 kDa, and loaded with the AKTA purifier 100 system. The XK16/100 chromatography column and Sephactyl S-200 from GE Company were used. The chromatography column was filled with a height of 95 cm. After eluting with 10 mM pH7.2 PB buffer containing 0.5% NaCl, the protein peaks were collected. The protein solution was rh-CRT-2, and the SDS-PAGE electrophoresis is shown in FIG 6.

### 1.5 Cloning and expression of rh-CRT-3

The amplification was carried out by PCR using pET28a-MCRT as template and pFcrt-3/pRcrt-3 as upstream and downstream primers, wherein an Nde I restriction site was introduced upstream and an Xho I restriction site was introduced downstream.
pFcrt-2 primer 5'-GTTCATATGGAACCGGCAGTGTATTTTAAG-3' (SEQ ID NO: 6)
pRcrt-3 primer 5'-GTGCTCGAGGTATGCGTAAATAGACGGGTC-3' (SEQ ID NO: 10)

The CRT gene sequence corresponding to rh-CRT-3 is set forth in SEQ ID NO: 11, and the protein sequence is set forth in SEQ ID NO: 12.

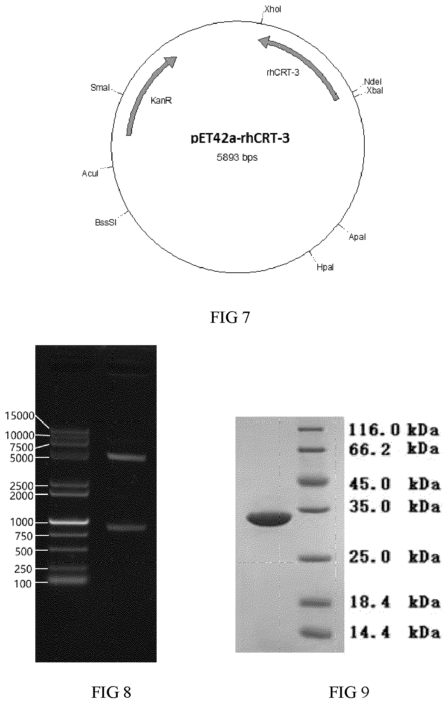

The Phusion high-fidelity DNA polymerase from Thermo Scientific was used for PCR with 30 cycles including 98°C for 10 seconds, 55°C for 30 seconds, and 72°C for 60 seconds, followed by incubation at 72°C for 10 minutes. The resulting PCR product (about 890 bp) was purified, digested with Nde I/Xho I, and inserted into the plasmid pET42a. The plasmid was then transformed into competent Escherichia coli TOP10, and positive clones were selected on LB medium containing kanamycin. The plasmid was extracted and sequenced for verification. The plasmid was named pET42a-rh-CRT-3. The plasmid map is shown in FIG 7 and the enzyme digestion results are shown in FIG 8. pET42a-rh-CRT-3 was transformed into competent BL21 (DE3). Positive clones were selected on LB plates containing kanamycin sulfate (50 µg/ml). The BL21 (DE3) positive clones obtained by transformation of pET42a-rh-CRT-3 were inoculated into 6ml LB liquid medium containing 50 µg/ml kanamycin sulfate, and cultured with shaking at 37°C, 220 RPM for about 3 hours. 2.0 ml bacterial liquid was inoculated in a 1.0 L Erlenmeyer flask containing 200 ml LB sterile medium with a final concentration of 50 µg/ml kanamycin sulfate, and cultured with shaking at 37°C, 220 RPM for 3 h until OD600 was about 1.0. Then, bacterial liquid was inoculated into six 5 L sterile Erlenmeyer flasks at a 1.0% inoculum, each of which contained 1.0 L LB medium, and cultured with shaking at 30°C, 220 RPM. When OD600 = about 0.8, 1.0 M IPTG was added to a final concentration of 0.5 mM, and cultured at a temperature adjusted to 28°C for 6 hours. Then the bacterial cells were collected by centrifugation.

### 1.6 Purification of rh-CRT-3 protein

10 g of the bacterial cells collected in 1.5 was added to 20 mM pH7.5 Tris-HCl buffer at a ratio of 1:20 (g/g) and subjected to ultrasonic lysis in an ice-water bath. The supernatant was collected by centrifugation, and loaded with an AKTA purifier 100 system. The XK16/20 chromatography column and 10 ml of Ni sepharose 6FF from GE Company were used. After washing with 20 mM pH7.5 Tris-HCl buffer containing 100 mM imidazole, eluting with 25 mM EDTA solution, the protein peaks were collected. The protein peaks were concentrated with an ultrafiltration centrifuge tube with a molecular weight cutoff of 5 kDa, and loaded with the AKTA purifier 100 system. The XK16/100 chromatography column and Sephactyl S-200 from GE Company were used. The chromatography column was filled with a height of 95 cm. After eluting with 10 mM pH7.2 PB buffer containing 0.5% NaCl, the protein peaks were collected. The protein solution was rh-CRT-3, and the SDS-PAGE electrophoresis is shown in FIG 9.

### Example 2

### 1. Experimental study on hair growth in mice

### 1.1 First Experiment

Experimental animals: C57BL/6 male mice, 6-7 weeks old, weighing (20±2) g, were provided by Beijing Vital River Laboratory Animal Technology Co. Ltd., with the animal license number of SCXK (Beijing) 2021-0006. They were kept in a 12 h light/12 h dark environment, with free access to food and water. The mice were divided into normal NC group (NC), model group (Model), positive control-minoxidil group (Minoxidil), positive control-finasteride group (Finasteride), experimental group A and experimental group B. The proteins were stored in a -70°C refrigerator and diluted with physiological saline when used. Experimental group A used rh-CRT-1, with a concentration of 0.21 mg/ml. Experimental group B used rh-CRT-2, with a concentration of 0.21 mg/ml. There were 6 mice in each group.

The main reagents used are as follows: 4% paraformaldehyde (Beijing Solarbio Technology Co., Ltd., China), isoflurane (Rayward, China), testosterone (Shanghai Standard Biotechnology Co., Ltd., China), paraffin sectioner (Thermo, USA), small animal anesthesia machine (Rayward, China), and inverted microscope (Olympus, Japan).

Modeling and drug administration: First, the androgenic alopecia model was established. One day before the modeling, the hair in the central part of the back of the experimental animal was removed with a skin preparation knife, and the prepared area was depilated with a depilatory cream. After 24 hours of depilation, the modeling agent (testosterone, 5.0 mg/mL) was subcutaneously injected into the neck of the mice in groups except the NC group, with a total dose of 0.2 mL/mouse. The mice in the NC group were injected with an equal amount of normal saline. After modeling, the mice were administrated with drugs. The NC group was injected with normal saline once a day, 0.15 mL/mouse each time. The experimental group A, experimental group B and positive control group were injected with modeling agent (testosterone), and administrated with drugs after 2 hours, and the dose of modeling agent was 0.2 mL/mouse. The experimental group A and experimental group B were injected on the back once a day, 0.15 mL/mouse each time. The positive control minoxidil group was applied 5% minoxidil externally, 0.01 mL/mouse, once a day. The positive control finasteride group was applied finasteride at a concentration of 13 µg /mL, 0.2 mL/mouse, once a day.

### Results:

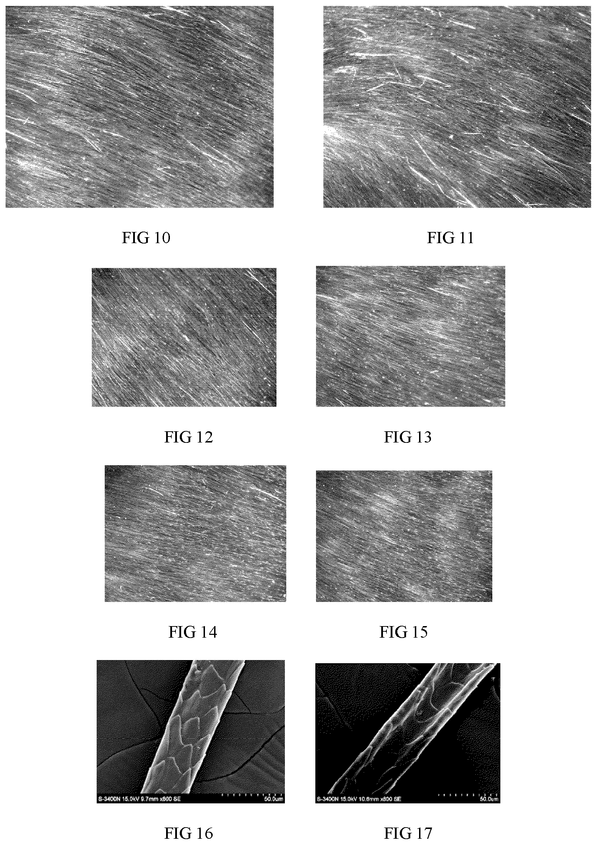

The experiment was carried out for 21 days, and the changes in hair in each group were observed by dermatoscope. The results are shown in FIG 10 to FIG 15. The normal control group had smooth hair, while the model group had frizzy hair. Compared with the model group, the hair of experimental group A and experimental group B was smoother. The positive control group, the minoxidil group and the finasteride group, had smooth hair.

The changes of hair shafts and hair cuticles were observed by scanning electron microscopy, and the results are shown in FIG 16 to FIG 21. The hair shafts of the normal control group were covered with overlapping scaly hair cuticles, and some hair shafts of the Model group appeared hollow. The hail cuticles of experimental groups A and B were arranged neatly, and the scaly hair cuticles of experimental group B were denser than those of group A. The hair cuticles of the positive control group - minoxidil group were scaly and arranged neatly, and the hair cuticles of the positive control group - finasteride group were arranged neatly.

### 1.2 Second experiment

Experimental animals: C57BL/6 male mice, 6-7 weeks old, weighing (20±2) g, were provided by Beijing Vital River Laboratory Animal Technology Co. Ltd., with the animal license number of SCXK (Beijing) 2021-0006. They were kept in a 12 h light/12 h dark environment, with free access to food and water. The mice were divided into normal control group (normal group, NC), model group (Model), experimental group C (rh-CRT-2, with a concentration of 0.35 mg/ml) including an injection group and an external application group, experimental group D (rh-CRT-3, with a concentration of 0.21 mg/ml) including an injection group and external application group, and positive control-minoxidil group (Minoxidil). The proteins were stored in a -70°C refrigerator and diluted with physiological saline when used. There were 6 mice in each group.

Modeling and drug administration: First, the hair loss model was established. One day before the modeling, the hair in the center of the back of the experimental animal was removed with a skin preparation knife, and the prepared skin area was depilated with a depilatory cream. After 24 hours of depilation, the normal control group was given 75% ethanol, and other groups were injected with testosterone solution (5 mg/mL) prepared with 75% ethanol, 0.01 mL/mouse. Two hours later, the injection groups of experimental group C and experimental group D were injected on the back, 0.15 mL/mouse, once a day, for 17 consecutive days; the external application groups of experimental group C and experimental group D were applied on the back, 0.15 mL/mouse, once a day, for 17 consecutive days. The normal control group and the model group were externally applied with physiological saline, once a day, 0.15 mL/mouse, for 17 consecutive days. During the drug administration process, except for the normal control group, all other groups were injected with the modeling agent (testosterone), and administrated with drugs after 2 hours. The hair condition was observed with dermatoscope, a color tester, and scanning electron microscope. After the experiment, the mouse skin was pathologically sectioned as follows: The skin pathological tissue was fixed in 4% paraformaldehyde, trimmed, dehydrated and transparent, and then embedded. The slices were cut with a thickness of 6 µm using a paraffin slicer, and further dewaxed, stained, dehydrated, transparent and sealed. The hair follicles in different areas were observed under an optical microscope, and the number of hair follicles was statistically analyzed.

Scanning electron microscopy observation: At the end of the experiment, hair shafts were randomly plucked from the newly formed hair area on the back of each group of mice. Three mice were selected from each group. The plucked hair shafts were fixed and the changes of hair shafts and hair cuticles were observed by scanning electron microscopy.

Hormone content in serum and skin tissue: At the end of the experiment, blood was collected from the mice's orbits, kept at room temperature for 30 min, centrifuged at 5,000 r/min for 10 min, and the serum was removed. At the end of the experiment, skin tissue was collected and stored in a -80°C refrigerator. During the experiment, the skin tissue was homogenized, and the changes in testosterone, dihydrotestosterone, and estradiol of the serum and skin tissue in each group were detected by enzyme-linked immunosorbent assay (ELISA).

Statistical analysis: All data were processed using SPSS 17.0 software. One-way ANOVA was used for comparisons among multiple groups. Data were expressed as mean ± standard deviation (*x̅*± s). P < 0.05 was considered statistically significant, and P > 0.05 was not considered statistically significant.

### Results:

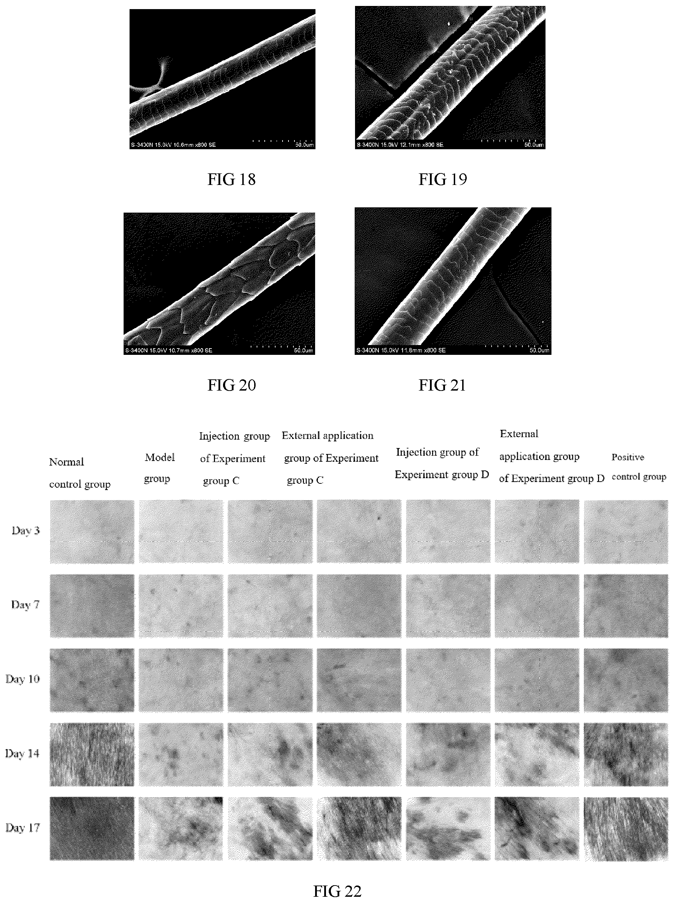

Under dermoscope, the entire back skin of the mice was photographed on the 3rd, 7th, 10th, 14th and 17th days of drug administration. The results are shown in FIG 22. On the 17th day of the experiment, hair grew on the back skin of the normal control group, while hair only grew on a small part of the skin of the model group. Both experimental group C and experimental group D promoted hair growth on the back of mice, among which the external application group of experimental group C promoted hair growth the most, followed by the injection group of experimental group D, the external application group of experimental group D and the injection group of experimental group C.

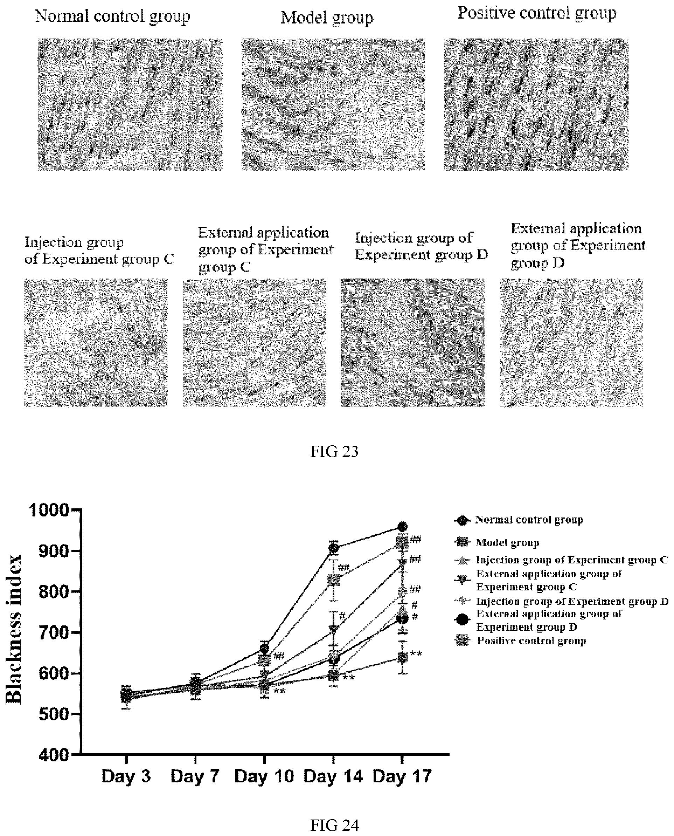

At the end of the experiment, after the back skin of each group was shaved and depilated, changes in the growth stage and quantity of back hair were observed under dermoscope. It was found that the hair in the normal control group was large and thick, while the hair in the model group was sparse, fine, and miniaturized. Compared with the model group, the hair in the external application group of experimental group C had increased number and became thicker, followed by the injection and external application group of experimental group D. The hair in the injection group of experimental group C was less and fine. The results are shown in FIG 23.

The changes in the blackness of the back skin of each group were observed using a color tester on the 7th, 10th, 14th and 17th days. The results are shown in FIG 24:
Day 7: There was no significant change in the blackness of the back skin of each group (P>0.05).

Day 10: Compared with the normal control group, the blackness of the back skin of the model group was significantly reduced (P<0.01). Compared with the model group, the blackness of the back skin did not change significantly in the drug group (P>0.05), and significantly increased in the minoxidil group (P<0.01).

Day 14: Compared with the normal control group, the blackness of the back skin of the model group was significantly reduced (P<0.01). Compared with the model group, the blackness of the back skin of the external application group of experimental group C was significantly increased (P<0.05).

Day 17: Compared with the normal control group, the blackness of the back skin of the model group was significantly reduced (P<0.01). Compared with the model group, the blackness of the back skin of the external application group of experimental group C and the injection group of experimental group D were significantly increased (P<0.01), and the blackness of the back skin of the injection group of experimental group C and the external application group of experimental group D were significantly increased (P<0.01).

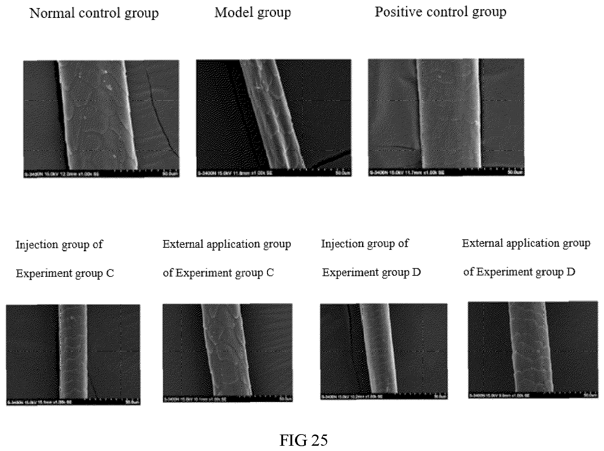

The changes in the hair of each group were observed under scanning electron microscopy, and the results are shown in FIG 25. The hair shafts of the normal control group were thick, and the surface of the hair shafts was covered by dense scaly hair cuticles arranged in layers. In the model group, the hair shafts were thin, the hair cuticles were sparse, and the surface was wrinkled. Compared with the model group, in the external application group of experimental group C, the hair shafts became significantly thicker, the hair shafts were smooth, and the superimposed scaly hair cuticles were dense; in the external application group of experimental group D, the hair shafts became thicker and the hair cuticles were dense. In the injection groups of experimental group C and experimental group D, although the hair shafts were thin, the hair cuticles were dense. The above results show that the hair of the external application group of experimental group C is significantly better than that of the model group in terms of hair shaft diameter and hair cuticle state, tending to be healthy.

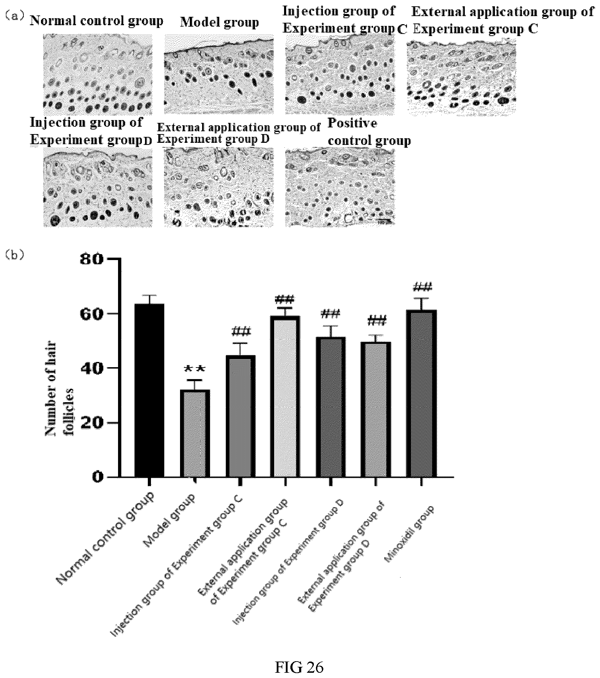

The pathological section results are shown in FIG 26. Compared with the normal control group, the number of hair follicles in the model group was significantly reduced, with statistical significance (P<0.01). Compared with the model group, experimental group C and experimental group D, both the external application group and the injection group, significantly promoted the expression of the number of hair follicles in androgen alopecia mice, with statistical significance (P<0.01). Among them, the external application group of experimental group C had the most significant effect on increasing the number of hair follicles. These results show that both experimental group C and experimental group D can improve the shrinkage and number reduction of hair follicles caused by testosterone and thus promote hair growth.

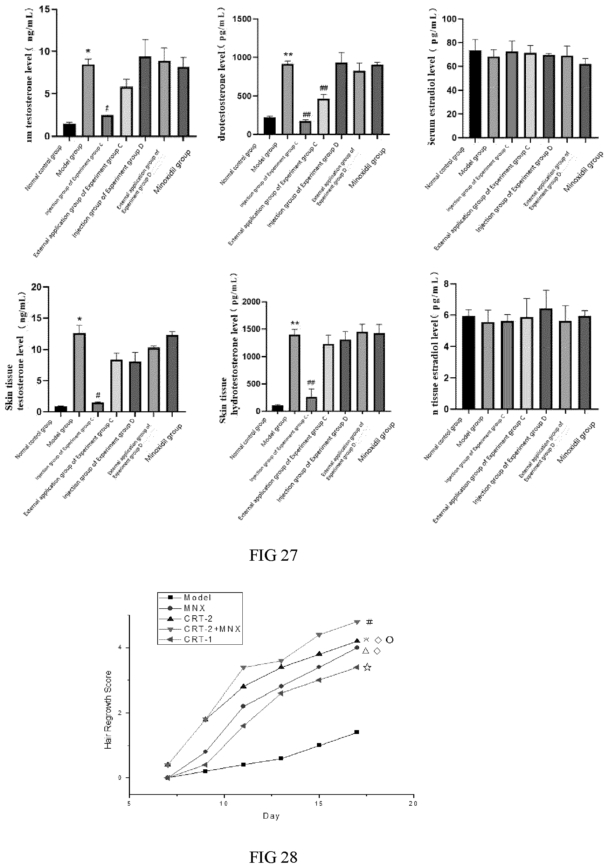

In order to observe the effects on testosterone, dihydrotestosterone and estradiol in the experimental groups, blood and skin tissue were collected at the end of the experiment to observe the changes in hormones in the serum and skin tissue. The results are shown in FIG 27 that:
Compared with the normal control group, the testosterone levels in the serum and skin tissue in the model group were significantly increased, with statistical significance (P<0.05). Compared with the model group, the testosterone levels in the serum and skin tissue in the injection group of experimental group C were significantly decreased (P<0.05), and there was no statistical significance in the serum and skin tissues of the other groups (P>0.05).

Compared with the normal control group, the serum and skin tissue dihydrotestosterone in the model group increased significantly, which was statistically significant (P < 0.01). Compared with the model group, the serum and skin tissue dihydrotestosterone in the experimental group C injection group decreased significantly (P < 0.01), and the serum dihydrotestosterone in the experimental group C topical group decreased significantly (P < 0.05), and there was no statistical significance in the serum and skin tissue of other groups (P > 0.05).

Compared with the normal control group, the estradiol levels in serum and skin tissue of the model group did not change (P<0.01). Compared with the model group, the estradiol levels in serum and skin tissue of other groups had no statistical significance (P>0.05), that is, there was no significant change in estradiol levels among the groups.

From the above results, it can be seen that the external application group of experimental group C can reduce the content of serum dihydrotestosterone, and its effect of promoting hair growth may be related to the reduction of dihydrotestosterone content.

### 1.3 Third experiment

In the first experiment, the experimental mice in experimental group A and experimental group B had smooth hair and neatly arranged hair cuticles. From the comparison of FIG 18 and FIG 19, the hair shafts of experimental group B (CRT-2, FIG 19) were thicker than those of experimental group A (CRT-1, FIG 18). Based on the optimization of the AGA (androgenic alopecia) model in mice, this experiment observed the hair growth effects of CRT-2 and CRT-1 in AGA model mice, as well as the effect of the combined use of CRT-2 and the positive control drug minoxidil.

### 1) Experimental animals:

C57BL/6N male mice, 6-7 weeks old, weighing (18-20) g, 50 mice, with the animal license number of SCXK (Beijing) 2021-0006, were kept in a 12h light/12h dark environment, with free access to food and water. Adaptive feeding was performed for about 1 week.

### 2) AGA model establishment and drug administration:

The hair in an area of about 2 cm×3 cm at the border of the spine was shaved using an electric shaver. The depilatory area was applied with 8% sodium sulfide solution. After 90±10s, the depilatory area on the back was cleaned with cotton balls dipped in water, the residual sodium sulfide was washed away with a washing bottle, and then the mice were wiped dry. Because the hair follicle cycle of mice was in the resting phase at this time, the mouse skin was pink. Mice were randomly divided into groups using Excel software, 10 mice in each group, and the modeling was established after 24 h and the drug was administered for 17 consecutive days. The AGA model was established by injecting dihydrotestosterone into the experimental mice. Dihydrotestosterone (DHT) solution was injected subcutaneously at multiple points every day, 100 µL/mouse (5mg/kg body weight), and then the drug was administered after 3 h of absorption. CRT-2 and CRT-1 protein solutions were applied externally. 100 µL of protein solution was applied, and 100 µl of minoxidil MNX was applied. The CRT and MNX combination group was first applied with CRT-2 protein solution and then MNX 2 hours later. The solvent formula comprised 50% ethanol, 20% propylene glycol, and 30% pure water. Grouping, modeling, and drug administration are shown in Table 1.

**Table 1**

| **Grouping** | **Hair Removal Method** | **Modeling** | **First drug administration** | **Second drug administration** |
|---|---|---|---|---|
| Model Group ( Model ) | Shaving + sodium sulfide | DHT injection 1 mg/mL, 100 µL | 3 hours later, apply 100 µL of normal saline | 2 hours later, apply 100 µL of solvent |
| CRT-2 Group | Shaving + sodium sulfide | DHT injection 1 mg/mL, 100 µL | 3 hours later, apply 100 µL of CRT-2 protein solution (0.35 mg/ml) | 2 hours later, apply 100 µL of solvent |
| Minoxidil group (MNX) | Shaving + sodium sulfide | DHT injection 1 mg/mL, 100 µL | 3 hours later, apply 100 µL of normal saline | 2 hours later, apply MNX (5 mg/ml) 100 µL |
| CRT-2 and Minoxidil | Shaving + | DHT injection | 3 hours later, | 2 hours later, |
| combination group (CRT-2+MNX) | sodium sulfide | 1 mg/mL, 100 µL | apply 100 µL of CRT-2 protein solution (0.35 mg/ml) | apply MNX (5 mg/ml) 100 µL |
| CRT-1 Group | Shaving + sodium sulfide | DHT injection 1 mg/mL, 100 µL | 3 hours later, apply CRT-1 (0.35 mg/ml) 100 µL | 2 hours later, apply 100 µL of solvent |

### 3) Hair growth score:

Scoring was based on the coverage of hair growth:
0 points: no hair growth, pink in the hair removal area;
1 point: gray in the hair removal area, hair coverage area E<20%;
2 points: black in the hair removal area, hair coverage area 20%<E<40%;
3 points: black in the hair removal area with a little hair growth, hair coverage area 40%<E<60%;
4 points: black in the hair removal area with some hair growth, hair coverage area 60%<E<80%;
5 points: black in the hair removal area, hair coverage area 80%<E<100%.

The skin color changes of mice were observed before administration every day. The hair removal time of mice was designated as Day 0, the first day of administration was designated as Day 1, and so on. The hair growth of each mouse was recorded and analyzed on Day 7, Day 9, Day 11, Day 13, Day 15, and Day 17.

### 4) Results:

On the 17th day after the administration of mice (Day17), compared with the model group, the hair of each group had significant growth, and there were statistical differences according to the preset score values. The results are shown in FIG 28. One-way analysis of variance was used for comparison among the groups. According to the score values, the results of the CRT-2 group were better than those of the CRT-1 group, and the combined CRT-2 and MNX group was better than the CRT-2 group alone and MNX group alone, indicating that CRT and MNX had a synergistic effect.
#Compared with the Model group, the hair growth score of the combined CRT-2 and MNX group was significantly increased (P<0.05);
-Compared with the Model group, the hair growth score of the CRT-2 group was significantly increased (P<0.05);
◊Compared with the Model group, the hair growth score of the MNX group was significantly higher (P<0.05);
☆Compared with the Model group, the hair growth score of the CRT-1 group was significantly higher (P<0.05); however, there was no significant difference between CRT-1 and MNX (P>0.05);
∘Compared with the CRT-1 group, the hair growth score of the CRT-2 group was significantly increased (P<0.05);
◊The hair growth score of the combined CRT-2 and MNX group was significantly different (P<0.05) compared with the CRT-2 group alone and the MNX group alone.

### 1.4 Fourth experiment

The idea and experimental process were basically the same as the third experiment, but the purpose of this experiment was to observe the hair growth effect of CRT-3 on AGA (androgen alopecia) model mice.

### 1) Experimental animals:

The experimental animals were the same as in the third experiment.

### 2) AGA model modeling and drug administration:

The AGA model was established in the same manner as the third experiment, and the administration was carried out as follows: The AGA model was established by injecting dihydrotestosterone into the experimental mice. Dihydrotestosterone (DHT) solution was injected subcutaneously at multiple points every day, 100 µL/mouse (5mg/kg body weight), and then the drug was administered after 3 h of absorption. CRT-3 and CRT-1 protein solutions were applied externally. 100 µL of protein solution was applied, and 100 µl of minoxidil MNX was applied. The solvent formula comprised 50% ethanol, 20% propylene glycol, and 30% pure water. Grouping, modeling, and drug administration are shown in Table 2.

**Table 2**

| **Grouping** | **Hair Removal Method** | **Modeling** | **First drug administration** | **Second drug administration** |
|---|---|---|---|---|
| Model Group ( Model ) | Shaving + sodium sulfide | DHT injection 1 mg/mL, 100 µL | 3 hours later, apply 100 µL of normal saline | 2 hours later, apply 100 µL of solvent |
| CRT-3 Group | Shaving + sodium sulfide | DHT injection 1 mg/mL, 100 µL | 3 hours later, apply 100 µL of CRT-3 protein solution (0.35 mg/ml) | 2 hours later, apply 100 µL of solvent |
| Minoxidil group (MNX) | Shaving + sodium sulfide | DHT injection 1 mg/mL, 100 µL | 3 hours later, apply 100 µL of normal saline | 2 hours later, apply MNX (5 mg/ml) 100 µL |
| CRT-1 Group | Shaving + sodium sulfide | DHT injection 1 mg/mL, 100 µL | 3 hours later, apply CRT-1 (0.35 mg/ml) 100 µL | 2 hours later, apply 100 µL of solvent |

### 3) Hair growth score:

The scoring criteria and scoring process were the same as those in the third experiment.

### 4) Results:

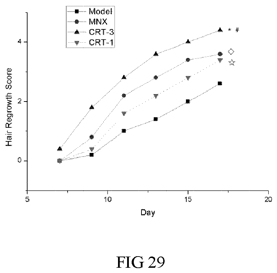

On the 17th day after the administration of mice (Day17), compared with the model group, the hair of each group had significant growth, and there were statistical differences according to the preset score values. The results are shown in FIG 29. One-way analysis of variance was used for comparison among the groups. According to the score values, compared with the Model group, the CRT-1, CRT-3, and MNX groups were all significantly higher; the results of the CRT-3 group were better than those of the CRT-1 group.
_{*}Compared with the Model group, the hair growth score of the CRT-3 group was significantly increased (P<0.05);
◊Compared with the Model group, the hair growth score of the MNX group was significantly higher (P<0.05);
☆Compared with the Model group, the hair growth score of the CRT-1 group was significantly higher (P<0.05); however, there was no significant difference between CRT-1 and MNX (P>0.05);
#Compared with the CRT-1 group, the hair growth score of the CRT-3 group was significantly increased (P<0.05);

Existing researchers generally believe that the full-length CRT protein should have better hair growth effects, but the results of the third and fourth experiments show that the truncated CRT fragments CRT-2 and CRT-3 have higher hair growth scores than the full-length CRT protein CRT-1.

It can be seen from the hair growth score after the combined use of CRT fragment CRT-2 and MNX that this combination significantly increases the hair growth score compared to the separate use of MNX and CRT-2 respectively, indicating that the combined use of CRT protein and MNX indeed produces a more excellent hair loss prevention effect.

## Claims

1. Application of calreticulin in preparing a product for hair growth, hair maintenance, hair protection or hair loss prevention.

2. The application according to claim 1, wherein the calreticulin is a protein of recombinantly expressed full-length CRT and having an amino acid sequence set forth in SEQ ID NO: 1, or a protein having a similarity of at least 85% to the amino acid sequence set forth in SEQ ID NO: 1 and having a similar function.

3. The application according to claim 1, wherein the calreticulin is a protein comprising a fragment of recombinantly expressed CRT and having an amino acid sequence set forth in SEQ ID NO: 9, or a protein having a similarity of at least 85% to the amino acid sequence set forth in SEQ ID NO: 9 and having a similar function.

4. The application according to claim 1, wherein the calreticulin is a protein comprising a fragment of recombinantly expressed CRT and having an amino acid sequence set forth in SEQ ID NO: 12, or a protein having a similarity of at least 85% to the amino acid sequence set forth in SEQ ID NO: 12 and having a similar function.

5. A recombinant protein related to hair growth, hair maintenance, hair protection or hair loss prevention, wherein the recombinant protein is rh-CRT-1, rh-CRT-2 or rh-CRT-3;
wherein rh-CRT-1 has an amino acid sequence set forth in SEQ ID NO: 1, rh-CRT-2 has an amino acid sequence set forth in SEQ ID NO: 9, and rh-CRT-3 has an amino acid sequence set forth in SEQ ID NO: 12.

6. A gene encoding the recombinant protein related to hair growth, hair maintenance, hair protection and hair loss prevention according to claim 5, wherein the gene has a nucleotide sequence encoding rh-CRT-1 set forth in SEQ ID NO: 2, a nucleotide sequence encoding rh-CRT-2 set forth in SEQ ID NO: 8, or a nucleotide sequence encoding rh-CRT-3 set forth in SEQ ID NO: 11.

7. A plasmid comprising the gene encoding the recombinant protein related to hair growth, hair maintenance, hair protection and hair loss prevention according to claim 6.

8. A vector comprising the gene encoding the recombinant protein related to hair growth, hair maintenance, hair protection and hair loss prevention according to claim 6 as an active ingredient.

9. Application of the recombinant protein according to claim 5, the gene according to claim 6, the plasmid according to claim 7 or the vector according to claim 8 in the manufacture of a medicament or medical device product for hair growth, hair maintenance, hair protection or hair loss prevention.

10. The application according to claim 9, wherein the medicament is utilized to improve the state of hair shaft and hair cuticle.

11. The application according to claim 9, wherein the medicament is utilized to increase the number of skin hair follicles.

12. The application according to claim 9, wherein the medicament promotes hair growth by reducing the content of androgen.

13. The application according to claim 12, wherein the androgen is dihydrotestosterone.

14. A product with functions of hair growth, hair maintenance, hair protection or hair loss prevention, comprising the recombinant protein according to claim 5, the gene according to claim 6, the plasmid according to claim 7 or the vector according to claim 8 as an active ingredient; wherein the product is utilized for at least one of the following:
a) improving hair smoothness, b) increasing hair volume, or c) promoting hair growth;
wherein the product is a medicament, an additive or an active ingredient.

15. A medicament with functions of hair growth, hair maintenance, hair protection or hair loss prevention, comprising the recombinant protein according to claim 5, the gene according to claim 6, the plasmid according to claim 7 or the vector according to claim 8 and a pharmaceutically acceptable adjuvant.

16. The medicament with functions of hair growth, hair maintenance, hair protection or hair loss prevention according to claim 15, wherein the medicament further comprises a pharmaceutical preparation that is utilized in combination, preferably minoxidil.

17. The medicament with functions of hair growth, hair maintenance, hair protection or hair loss prevention according to claim 15, wherein the adjuvant is selected from the group consisting of a diluent, excipient, filler, binder, wetting agent, disintegrant, absorption promoter, surfactant, adsorption carrier, lubricant, and a mixture thereof.

18. The medicament with functions of hair growth, hair maintenance, hair protection or hair loss prevention according to claim 15, wherein the medicament is introduced into a body tissue by injection, spraying, penetration, absorption, or a physical or chemical method; or the medicament is introduced into a body after being mixed or encapsulated by other substances.

19. The medicament with functions of hair growth, hair maintenance, hair protection or hair loss prevention according to claim 15, wherein the medicament is an external preparation.

20. Application of calreticulin in the manufacture of a medicament for treating acne, pimples or alopecia areata.

21. The application according to claim 20, wherein the calreticulin CRT is a protein of recombinantly expressed full-length CRT and having an amino acid sequence set forth in SEQ ID NO: 1, or a protein having a similarity of at least 85% to the amino acid sequence set forth in SEQ ID NO: 1 and having a similar function.

22. The application according to claim 20, wherein the calreticulin CRT is a protein comprising a fragment of recombinantly expressed CRT and having an amino acid sequence set forth in SEQ ID NO: 9, or a protein having a similarity of at least 85% to the amino acid sequence set forth in SEQ ID NO: 9 and having a similar function.

23. The application according to claim 20, wherein the calreticulin CRT is a protein comprising a fragment of recombinantly expressed CRT and having an amino acid sequence set forth in SEQ ID NO: 12, or a protein having a similarity of at least 85% to the amino acid sequence set forth in SEQ ID NO: 12 and having a similar function.
